(19) Europäisches Patentamt

European Patent Office

Office européen des brevets

(11) **EP 4 542 568 A1**

(12) **EUROPEAN PATENT APPLICATION**

(43) Date of publication:
**23.04.2025 Bulletin 2025/17**

(21) Application number: **23204906.4**

(22) Date of filing: **20.10.2023**

(51) International Patent Classification (IPC):
**G16H 40/63** $^{(2018.01)}$     **A61M 16/00** $^{(2006.01)}$
**G16H 20/40** $^{(2018.01)}$

(52) Cooperative Patent Classification (CPC):
**G16H 20/40; A61M 16/00; G16H 40/63**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventor: **ZHAO, Xinying
Eindhoven (NL)**

(74) Representative: **Philips Intellectual Property &
Standards
High Tech Campus 52
5656 AG Eindhoven (NL)**

(54) **PROVISION OF VENTILATION INFORMATION**

(57)    A mechanism for displaying a first visual representation of a first ventilation parameter and a second visual representation of a second ventilation parameter. A user interface is controlled to provide the first and second visual representations. The second visual representation is positioned so as to partially overlap the first visual representation.

**FIG. 2**

**Description**

FIELD OF THE INVENTION

The present invention relates to the field of medical ventilation, and in particular, to the use of medical ventilators.

BACKGROUND OF THE INVENTION

There is an increasing use of mechanical ventilators to perform medical ventilation. ventilation parameters, respiratory-related physiological parameters, and/or errors detected during the course of mechanical ventilation. It is important that an operator of the mechanical ventilator (e.g., a clinician, caregiver or the ventilated subject) is able to perceive the ventilation information. To this end, it is typical to provide a visual representation of the ventilation information, or a portion of the ventilation information.

Examples of ventilation parameters, for which ventilation information may be displayed, include usage duration, leakage, mask fit, tidal volume, respiratory rate, blood saturation level, apnea hypopnea index (AHI) and the percentage of breaths triggered and cycled by the patient.

Ventilation information is normally represented in a numeric format, together with a with textual explanation and/or identification of each ventilation parameter. It is known to provide simple graphical visualizations, such as horizontal/vertical histograms or fan diagrams, to represent changes of a certain ventilation parameter.

There is an ongoing desire to improve the display of ventilation information to an operator of a mechanical ventilator.

SUMMARY OF THE INVENTION

The invention is defined by the claims.

According to examples in accordance with an aspect of the invention, there is provided a computer-implemented method of controlling the display of ventilation information.

The computer-implemented method comprises receiving ventilation information comprising at least one value for each of at least two ventilation parameters for a subject undergoing mechanical ventilation, wherein the at least two ventilation parameters comprise a first ventilation parameter and a second ventilation parameter; controlling a user interface to provide a first visual representation of the at least one value of the first ventilation parameter; and controlling the user interface to provide a second visual representation of the at least one value of the second ventilation parameter, wherein the second visual representation at least partially overlays the first visual representation.

The proposed approach thereby resolves conflicting technical problems of how to present more than one piece of ventilation information on a same screen or user interface of a restricted size. The present disclosure proposes to overlap the visual representations for different pieces of ventilation information to facilitate the provision of multiple pieces of ventilation information in a smaller screen space. The ventilation parameters may include ventilator related parameters (e.g., usage duration, leakage, humidity, and so on) and/or patient related parameters (for example, SPOz, apnea hypopnea index (AHI), and so on).

In some examples, the second visual representation wholly overlays the first visual representation. This approach further improves the compactness of the display of ventilation information without generating adverse influence on the display for the relevant information.

In some examples, a textural appearance of the first visual representation changes as the at least one value for the first ventilation parameter changes; and/or a textural appearance of the second visual representation changes as the at least one value for the second ventilation parameter changes. Use of textural appearance changes (i.e., changes in texture) facilitates the indication of changes to ventilation parameter(s) without the reliance upon color changes (and without needing to change the shape or transparency of the visual representation). This advantageously provides an indicator of change of a ventilation parameter that is perceptible by a colorblind individual without the need to modify a shape or structure of the visual representation which would necessitate significant and resource-intensive modification(s) to the display.

The first visual representation and/or the second visual representation may be an animated visual representation.

In some examples, the at least two ventilation parameters further comprise a third ventilation parameter; and the second visual representation is configured to provide a visual representation of the at least one value of the second ventilation parameter and the at least one value of the third ventilation parameter.

In some examples, the second visual representation is configured to sequentially provide the visual representation of the at least one value of the second ventilation parameter and the at least one value of the third ventilation parameter, such that the visual representation of the at least one value of the second ventilation parameter is not provided at a same time as the visual representation of the at least one value of the third ventilation parameter.

In some examples, the first ventilation parameter or the second ventilation parameter is a time remaining ventilation parameter having a value that indicates a recommended remaining ventilation time for the subject, such that one of the first or second visual representation is associated with time remaining ventilation parameter; and one or more visual properties of the visual representation associated with the time remaining ventilation parameter changes responsive to the value that indicates a recommended remaining ventilation time for the subject.

The one or more visual properties may comprise one or more of: a transparency; a texture; a color; a size; and/or a number of icons.

The one or more visual properties may comprise a size of the visual representation, wherein the size of the visual representation associated with the time remaining ventilation parameter decreases proportionally with a decrease in the value of the recommended remaining ventilation time for the subject.

In some examples, the ventilation information further comprises, for the first and/or second ventilation parameter, time information identifying a time elapsed since a last recorded value for said ventilation parameter, and the computer-implemented method further comprises: when the time information identifies a time elapsed since the last recorded value for the first ventilation parameter, controlling a visual property of the first visual representation responsive to the time information; and/or when the time information identifies a time elapsed since the last recorded value for the second ventilation parameter, controlling a visual property of the second visual representation responsive to the time information.

In some examples, when the time information identifies a time elapsed since the last recorded value for the first ventilation parameter, increasing a transparency of the first visual representation as the time elapsed since a last recorded value for the first ventilation parameter increases; and/or when the time information identifies a time elapsed since the last recorded value for the second ventilation parameter, increasing a transparency of the second visual representation as the time elapsed since a last recorded value for the second ventilation parameter increases.

In some embodiments, the at least one value for the first ventilation parameter comprises a numerical measure of the first ventilation parameter; and a hue angle of a color of the first visual representation changes proportionally to the numerical measure of the first ventilation parameter.

In some examples, at least one of the first visual representation and second visual representation is interactive. The computer-implemented method may comprise, responsive to a user interaction with the first visual representation and/or the second visual representation: identifying the first and/or second visual representation with which the user has interacted as an interacted visual representation; and providing a further visual representation of at least one: an explanation of the ventilation parameter associated with the interacted visual representation; trouble-shooting guidance for the ventilation parameter associated with the interacted visual representation; usage guidance for the ventilation parameter associated with the interacted visual representation; and/or historic parameter information for the ventilation parameter associated with the interacted visual representation.

In some examples, the step of providing the further visual representation comprises: obtaining historic parameter information for the ventilation parameter associated with the interacted visual representation; and controlling the user interface to provide a display of the historic parameter information.

In some examples, the historic parameter information identifies, for the ventilation parameter associated with the interacted visual representation, one or more values of the ventilation parameter and, for each value, a historic time period in which the value was captured or recorded; the step of controlling the user interface to provide a display of the historic parameter information comprises: associating different portions of the user interface with different historic time periods; and controlling, for each portion of the user interface, a visual representation in said portion of the user interface responsive to any values of the ventilation parameter associated with the historic time period of said portion of the user interface.

In some examples, the one or more values of the ventilation parameter associated with the interacted visual representation are numeric measures, the step of controlling, for each portion of the user interface, a visual representation in said portion of the user interface comprises controlling a visual property of the visual representation in said portion of the user interface responsive to a sum of all values of the ventilation parameter associated with the historic time period of said portion of the user interface.

There is also provided a computer program product comprising computer program code means which, when executed on a computing device having a processing system, cause the processing system to perform all of the steps of any herein disclosed method.

There is also provided a user interface system comprising: a user interface; and a processing system configured to: receive ventilation information comprising at least one value for each of at least two ventilation parameters for a subject undergoing mechanical ventilation, wherein the at least two ventilation parameters comprise a first ventilation parameter and a second ventilation parameter; control the user interface to provide a first visual representation of the at least one value of the first ventilation parameter; and control the user interface to provide a second visual representation of the at least one value of the second ventilation parameter, wherein the second visual representation at least partially overlays the first visual representation.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a user interface system;
Fig. 2 illustrates a first example display;
Fig. 3 illustrates a second example display;
Fig. 4 illustrates a third example display;
Fig. 5 illustrates a fourth example display;
Fig. 6 illustrates a fifth example display;
Fig. 7 illustrates a sixth example display; and
Fig. 8 is a flowchart illustrating a proposed method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for displaying a first visual representation of a first ventilation parameter and a second visual representation of a second ventilation parameter. A user interface is controlled to provide the first and second visual representations. The second visual representation is positioned so as to partially overlap the first visual representation.

In the context of the present disclosure, the term "ventilation" refers to medical ventilation or mechanical ventilation, i.e., artificial ventilation for controlling the respiration or breathing of an individual.

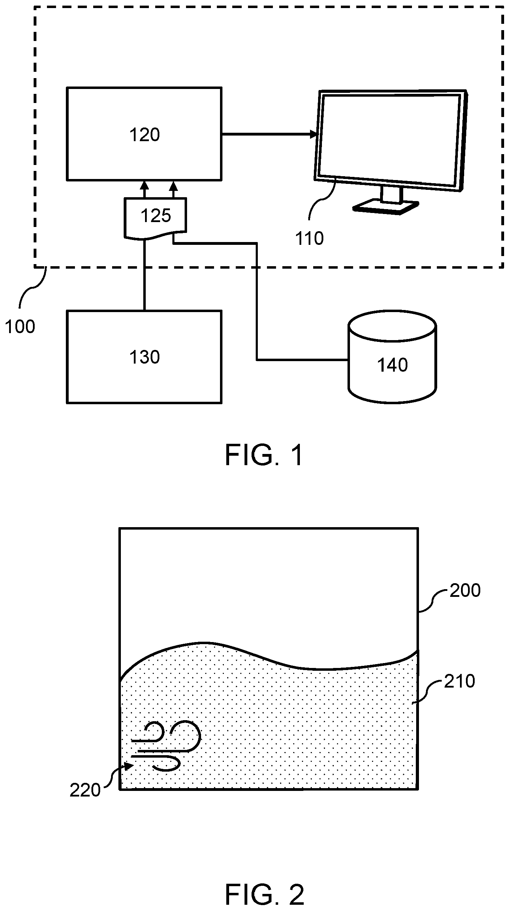

Fig. 1 illustrates a user interface system 100 in which proposed embodiments can be employed. The display system 100 comprises a user interface 110 (comprising a display) and a processing system 120.

The display system 100 is configured for displaying ventilation information 125 of a medical subject, e.g., generated by a ventilation system 130. The processing system 120 may obtain the ventilation information 125 directly from the ventilation system 130 and/or from a memory or storage system 140 (e.g., which stores information generated by the ventilation system 130).

The ventilation information 125 comprises at least one value for at least one ventilation parameter. A ventilation parameter is any parameter that can be produced responsive to monitoring by a ventilation system. This includes any parameter that relates to the use of the ventilation system and/or the effects of the use of the ventilation system. Other ventilation parameters include recommended actions responsive to a monitored value (e.g., a recommendation on whether to increase or reduce airflow). Example parameters include usage duration, humidity, blood saturation level, leakage, mask fit, tidal volume, respiratory rate, blood saturation level, apnea hypopnea index (AHI) and the percentage of breaths triggered and cycled by the patient.

Approaches for facilitating the control of a user interface by a processing system are well known in the art, and typically comprise the processing system generating display data that is output at the user interface.

The processing system 120 is configured to obtain the ventilation information and control the user interface to provide a display of the ventilation information.

The present disclosure provides a variety of mechanisms and approaches for improved visualization of the ventilation information. In particular, proposed approaches facilitate the provision of a greater amount of ventilation information on a same size display compared to previous approach. The processing system may use any one or more of the proposed approaches to control the display of information at the user interface.

Example approaches are disclosed in the context of a set of example displays. Each display may represent a display provided by the user interface under the control of the processing system. Approaches for controlling a user interface to provide a desired display are well known in the art, and are not discussed in detail for the sake of conciseness.

Fig. 2 illustrates an example display 200 provided by a user interface employing a herein proposed approach. The display 200 provides a first visual representation 210 of a first ventilation parameter and a second visual representation 220 of a second ventilation parameter.

For the example display 200, the first ventilation parameter is relative humidity of gas provided to the medical subject during ventilation (hereafter: humidity). This parameter can be sensed using a humidity sensor positioned in a mask, outflow valve or hose of the ventilation system.

One or more visual properties of the first visual representation changes responsive to changes in the first ventilation parameter (e.g., humidity here). In the illustrated example, the first visual representation 210 may be configured to fill more of the display 200 with texture and/or color as the value for humidity increases to provide a visual indicator to a view of the display 200 of the humidity. Thus, the size of the first visual representation may change responsive to changes in humidity.

It will be apparent that the first visual representation 210 may occupy the entirety of the display 200 (e.g., for extremely high humidity values).

For the example display 200, the second ventilation parameter is a leakage estimation, e.g., being a measure of (unintentional) leakage of gas provided to the medical subject during ventilation. The leakage may be estimated by a built-in software of the ventilation system, e.g., taking into account the type of mask used, pressure, leakage compensation and so on. Approaches are known to the appropriately skilled person.

Similarly to the first visual representation, one or more visual properties of the second visual representation change responsive to changes in the leakage (estimation). By way of example, the size of the second visual representation may change, the type of icon may change, the number of icons may change and so on.

The second visual representation 220 is configured to at least partially overlay the first visual representation 210. In the illustrated example, the second visual representation 220 wholly overlays the first visual representation 210. By overlapping the two visual representations, the amount of information that can be provided by a single display is increased, thereby resolving conflicting technical requirements of how to provide multiple visual representations of information without increasing a screen size.

By way of example only, the second visual representation may be configured to change or adjust the icon responsive to a leakage in a past predetermined period of time (e.g., a past two hours). For instance, when a leakage of more than 24L/min occurs fewer than a predetermined number of time (e.g., less than 3 times) in the past predetermined period of time, then no icon may be shown; when a leakage of more than 24L/min occurs more than or equal to a predetermined number of time (e.g., $\geq$ 3 times) in the past predetermined period of time, then a first icon may be shown (e.g., as illustrated by Fig. 2); and when a leakage of more than 24L/min occurs more than a second predetermined number of time (e.g., more than 5 times) in the past predetermined period of time, then a second, different icon may be shown.

To improve efficiency of data visualization, the second visual representation wind can be animated periodically or in a motion that represents the speed and/or volume of leakage. This helps convey the information of serious leakage, e.g., a leakage requiring a mask adjustment. A moving or animated visual representation is more noticeable than a static visual representation.

To further improve visualization of the ventilation information, the texture of one or both of the first and second visual representation may change responsive to changes in the value of the first and second ventilation parameter respectively.

Thus, for instance, the texture of the first visual representation may change responsive to changes in the first ventilation parameter. This advantageously allows for changes in the appearance of the first visual representation that allow colorblind individuals to perceive the change without affecting the size, shape or screen occupation of the first visual representation.

By way of example only, if the first visual representation changes responsive to a change in humidity, then lower values of humidity may result in the first visual representation having a texture similar to a desert or sand, whereas higher values of humidity may result in the first visual representation having a texture similar to grass, with yet high values of humidity resulting in the first visual representation having a texture similar to water.

A wide variety of other textures and the like will be readily apparent to the person skilled in the art. For instance, a number of stripes, dots, stippling and/or other textural parameters may change responsive to changes in the ventilation parameter associated with the visual representation.

To improve the visualization of the ventilation information, the color (hue) of at least one visual representation may have responsive to changes in the corresponding ventilation parameter.

For instance, if the value(s) for the ventilation parameter is a numeric measure, then the hue angle of the color of the visual representation may change proportionally to the magnitude of the numeric measure.

Mathematically, this can be expressed as:

$$Icon_{ha} = a_n VP + b_n \qquad (1)$$

where $Icon_{ha}$ is a hue angle of the visual representation (i.e., the icon), VP is the value of the ventilation parameter, $a_n$ is a

first coefficient and $b_n$ is a second (biasing) coefficient.

In an alternative approach, the value of the ventilation parameter is compared to one or more number ranges. Each number range may be associated with a different hue angle or range of hue angles (hue angle range). Responsive to the value of the ventilation parameter falling within a particular number range, the hue angle of the corresponding visual representation is set to be a hue angle within the hue angle range associated with the number range.

Where a number range is associated with a hue angle range, then the hue angle of the corresponding visual representation may be determined proportional within the hue angle range to the location of the value of the ventilation parameter in the number range. Conceptually, this can be expressed as:

$$\frac{Icon_{ha} - har_l}{har_h - har_l} = \frac{VP - nr_l}{nr_h - nr_l} \tag{2}$$

where VP represents the value of the ventilation parameter, $Icon_{ha}$ represents the hue angle of the icon, $har_l$ represents the lower bound of the hue angle range, $har_h$ represents the upper bound of the hue angle range, $nr_l$ represents the lower bound of the number range and $nr_h$ represents the upper bound of the hue angle range.

As a working example, consider a scenario in which the first visual representation represents a value of humidity RH (relative humidity). The first visual representation is configured to change texture and color responsive to the value of humidity (although, of course, one of these visual property changes may be omitted in variations).

In this working example, when $0 < RH \leq 40\%$, the first visual representation may be is mainly yellow in color with texture of desert and short and flat symbols indicating lack of water. The hue angle may vary between 30° and 60° with increasing values of RH to visually indicate increased water saturation. In this configuration, the first visual representation may prompt the operator to turn on/up the humidifier in the ventilator, add water to the water tank to ensure the normal operation of the humidifier, or even turn on/up the humidifier in the environment (if applicable), until the humidity level reaches a desired or normal.

In this working example, when $40\% < RH \leq 60\%$, the first visual representation may be mainly green in color with texture of grass and/or a medium symbol indicating sufficient water. The hue angle may vary between 75° and 165°. In this configuration, the first visual representation may suggest the humidity level is in normal range and no change is needed.

In this working example, when $60\% < RH \leq 100\%$, the first visual representation may be mainly blue in color with texture of seawater and high symbol indicating excessive water. The hue angle may vary between 180° and 240°. In this configuration, the first visual representation may prompt the operator to turn down/off the humidifier in the ventilator and in the environment (if applicable), turn on the heating circuits or even turn on a dehumidifier in the environment (if applicable), until the humidity level is in normal range.

This approach merely provides one working example of how a texture and/or color of a visual representation may change responsive to changes of a ventilation parameter, and other examples will be readily apparent to the skilled person.

To improve the visualization of the ventilation information, the first visual representation and/or the second visual representation may be an animated visual representation. An animated visual representation is more noticeable than a non-animated visual representation, drawing the viewer's attention to the particular visual representation.

In some examples, the animation is responsive to a value of the ventilation parameter associated with the visual representation. Thus, the animation may provide additional detail to a viewer of the visual representation. By way of example only, a visual representation representing a leakage could be animated to represent the speed and volume of leakage (e.g., when above threshold). Other examples will be apparent to the skilled person.

Thus, in some examples, one or more visual representations are configured to become animated only when a value for the corresponding ventilation parameter meets one or more predetermined conditions (e.g., clinically undesirable conditions). This relies upon movement to draw an observer's attention to the existence of the predetermined condition(s), e.g., the existence of clinically undesirable conditions. By way of example, a visual representation may become animated when a numeric value of the corresponding ventilation parameter breaches a predetermined threshold (e.g., a clinically undesirable threshold). Otherwise, the visual representation may be non-animated, e.g., static.

In some examples, each visual representation in an animated set of visual representations may be associated with a plurality of different ventilation parameters. The animation of each visual representation in the animated set may be animated so as to switch or change which ventilation parameter is visually represented and how one ventilation parameter is visually represented.

Consider a scenario in which the second visual representation is in the animated set of visual representations. In this scenario, the at least two ventilation parameters may further comprise a third ventilation parameter and the second visual representation may be configured to provide a visual representation of the at least one value of the second ventilation parameter and the at least one value of the third ventilation parameter.

In particular, the second visual representation may be configured to sequentially provide the visual representation of the at least one value of the second ventilation parameter and the at least one value of the third ventilation parameter, such that the visual representation of the at least one value of the second ventilation parameter is not provided at a same time as the visual representation of the at least one value of the third ventilation parameter.

This can be achieved, for instance, by the second visual representation appearing to flip between displaying the value(s) of the second ventilation parameter and the value(s) of the third ventilation parameter. Other approaches could be used, e.g., fading between the two ventilation parameters or the like.

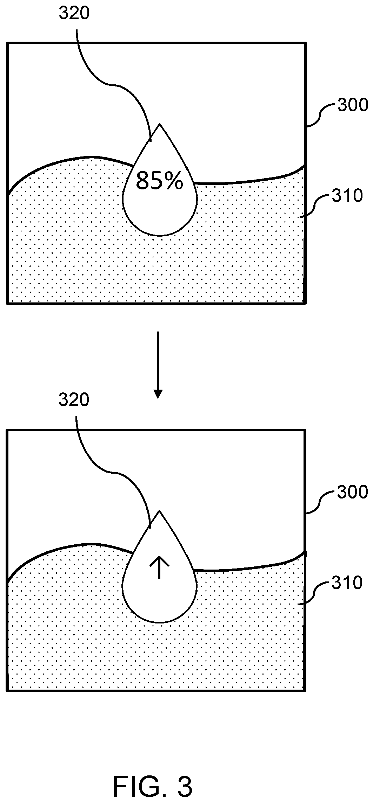

Fig. 3 conceptually illustrates an example animated visual representation 320 on a display 300. The animated visual representation 320 is here a second visual representation that partially overlays a first visual representation 310 of the display. The first visual representation 310 indicates a value for a first ventilation parameter, e.g., humidity.

The second visual representation 320 is configured to switch between displaying a value of a second ventilation parameter (here: blood saturation level) and a third ventilation parameter (here: recommended oxygen supply change). In this way, the display is able to switch between providing information on two ventilation parameters.

The second and third ventilation parameters may be related. For instance, the third ventilation parameter may be a recommendation action or detailed information for the second ventilation parameter. Approaches for determining recommended actions based on values of a ventilation parameter are well known in the art.

To improve the visualization of the ventilation information, one or more visual properties of a visual representation may change responsive to an elapsed time since the last recorded value for the corresponding ventilation parameter. This provides an indicator to an operator of the ventilation system as to a reliability and/or age of the displayed value for the ventilation parameter, which may influence a treatment decision.

The elapsed time may be readily calculated by, for instance, comparing a current time to a time recorded in a timestamp for the last recorded value of the corresponding ventilation parameter.

One example of a suitable visual property is a transparency of the visual representation. In particular, a transparency of the visual representation may increase as the time since the last recorded value for the corresponding ventilation parameter increases.

Another example of a suitable visual property is a color fill percentage of the visual representation. A color fill percentage indicates a percentage or proportion of the visual representation that is filled with a color (e.g., is not transparent). In particular, a color fill percentage of the visual representation may decrease as the time since the last recorded value for the corresponding ventilation parameter increases.

Another example of a suitable visual property is a flickering of the visual representation. A flickering indicates a presence of flicker and/or speed at which visual representation flickers (e.g., temporarily changes transparency, opacity and/or color).

A wide variety of other suitable visual properties may be used or modified responsive to a time since the last recorded value for the corresponding ventilation parameter increases.

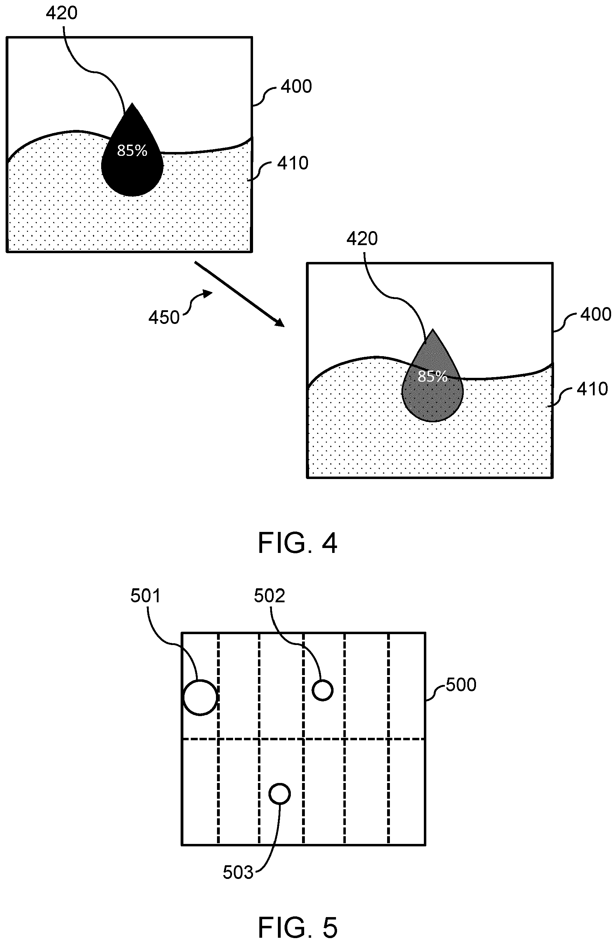

Fig. 4 conceptually illustrates an example visual representation 420 on a display 400 that changes responsive to an elapsed time since the last recorded value for the corresponding ventilation parameter. Here, the example visual representation 420 is a second visual representation that partially overlays a first visual representation 410 of the display. The first visual representation 410 again indicates a value for a first ventilation parameter, e.g., humidity.

Here, the second visual representation represents a value for a blood oxygen level. A transparency of the second visual representation increases (i.e., the second visual representation becomes less opaque) as the time since the last recorded value for blood oxygen level increases (as indicated by the arrow 450).

One approach for determining a transparency $Icon_{transparency}$ for a visual representation is as follows:

$$Icon_{transparency} = \frac{100(T_{now} - T_{tested})}{T_{period}} \qquad (6)$$

where $T_{npw}$ represents a current point in time, $T_{tested}$ represents the time at which the last recorded value for the ventilation parameter was recorded and $T_{period}$ represents a predefined period of time, where the predefined period of time may represent a maximum acceptable time since last recording of the ventilation parameter for making a clinical decision.

To improve visualization of the ventilation information, the color fill percentage of one or both of the first and second visual representations may change responsive to changes in the value of the first and second ventilation parameter respectively. A color fill percentage indicates a percentage or proportion of the visual representation that is filled with a color (e.g., is not transparent).

To improve the visualization of the ventilation information, one or more of the visual representations may be interactive. In particular, interacting with a visual representation may cause a further visual representation to be provided. The further visual representation may, for instance, provide an explanation of the ventilation parameter associated with the interacted visual representation; trouble-shooting guidance for the ventilation parameter associated with the interacted visual representation; usage guidance for the ventilation parameter associated with the interacted visual representation; and/or historic parameter information for the ventilation parameter associated with the interacted visual representation.

As a working example, the ventilation parameter associated with an interactive visual representation may be associated (e.g., via a database mapping) with supportive information comprising a particular explanation, trouble-shooting guidance and/or usage guidance. Responsive to an interaction, this supportive information may be displayed in the form of a further visual representation to aid the operator of the ventilation system.

As another example, the ventilation parameter may be associated with historic parameter information. Providing the further visual representation may comprise controlling the user interface to provide a display of the historic parameter information.

In some examples, the historic parameter information identifies, for the ventilation parameter, one or more values of the ventilation parameter and, for each value, a historic time period in which the value was captured or recorded. Thus, each value is associated with a historic time period. The historic parameter information may comprise, for instance, a timestamp for each value identifying when the value was captured or recorded, as a timestamp will indicate the time period. Each historic time period may have a predefined or predetermined length (e.g., 1 hour or 2 hours).

When an interaction with an interactive visual representation occurs, the further visual representation may be displayed by associating different portions of the user interface with different historic time periods; and controlling, for each portion of the user interface, a visual representation in said portion of the user interface responsive to any values of the ventilation parameter associated with the historic time period of said portion of the user interface.

In this way, different portions of the display represent different historic time periods. Information responsive to the or any value(s) for the ventilation parameter are displayed in the portion corresponding to their historic time period.

Thus, for instance, if a first portion represents a time period of between 2 hours and 1 hour in the past, and a second portion represents a time period of between 1 hour in the past and the present - then any values obtained between 1 hour and 2 hours ago are visually represented in the first portion, and any values obtained less than 1 hour ago are visually represented in the second portion.

In one example, a visual property of the visual representation in each portion of the display represents or is responsive the largest value of the historic time period. In another example, a visual property of the visual representation in each portion of the display represents or is responsive the smallest value of the historic time period. In another example, a visual property of the visual representation in each portion of the display represents or is responsive the average value of the historic time period.

As another example, a visual property of the visual representation in each portion may be responsive to a sum of all values associated with the historic time period of that portion. For instance, a size of an icon may be responsive to the sum of all values associated with the historic time period of said portion. Fig. 5 illustrates a display 500 after a further visual representation has been displayed (e.g., responsive to an interaction with an interactive visual representation for one ventilation parameter). The further visual representation may replace all visual representations previously provided in the display.

As illustrated, the display 500 is conceptually divided into a plurality of different portions. Each portion is associated with a different period of time. The further visual representation comprises one or more icons 501, 502, 503 or symbols. The size of each icon or symbol is responsive to the sum of all values (for the corresponding ventilation parameter) in that historic time period. Of course, icons are only present in those portions representing historic time periods associated with any value of the ventilation parameter.

In the illustrated example, the display is divided into 12 portions, e.g., each representing a respective time period having a length of 1 hour or 2 hours. The time period represented by each portion may immediately abut the time period represented by another portion. Of course, the display may be divided into any number of portions, e.g., more than 12 portions. It is not essential that each portion be rectangular. Rather, portions may take any shape or combination of shapes.

Other visual properties that may be varied responsive to the sum of all values (associated with that portion) include a color, a texture, a number of icons and so on.

Previously described embodiments provide different display mechanisms for providing visual representation(s) of ventilation parameters, to improve the visualization of the ventilation information.

A number of examples of ventilation parameters and their display mechanisms have been provided in the preceding description, including humidity, leakage and blood saturation levels. Further examples of ventilation parameters, and approaches for displaying the same, are hereafter provided. Other examples will be well known to the person skilled in the art.

One example of a ventilation parameter is a time remaining ventilation parameter. Accordingly, in some examples, the first ventilation parameter or the second ventilation parameter is a time remaining ventilation parameter. A value of the time remaining ventilation parameter indicates a recommended remaining ventilation time for the subject (undergoing ventilation). In this one, one of the first or second visual representations is associated with a time remaining ventilation parameter.

One or more visual properties of the visual representation associated with the time remaining ventilation parameter may change responsive to the value that indicates a recommended remaining ventilation time for the subject. By way of example, the number of icons in a visual representation may change responsive to the recommended remaining ventilation time. As another example, the size of one or more icons may change responsive to the recommended remaining ventilation time.

It is conventional for a ventilator manufacturer to provide the total usage time in a day as well as the usage history for users. However, it is unclear for lay-users of the ventilation system to understand how long they should use the ventilator. It is therefore proposed to provide the operator of the ventilation system with an indication of the remaining recommended time to aid them in performing ventilation appropriately and correctly. Hence, a visualization reflecting the related data timeliness is provided to facilitate the treatment and clinical decision-making for both patients and clinician. One approach for calculating the recommended remaining ventilation time is to make use of timestamps recorded by the ventilator system (e.g., recorded in one or more logs), which includes the timestamps of turning on and off the ventilator in a certain calendar day (e.g., in a 24 hour period, e.g., from 7:00 am to 7:00am the next day), according to the time zone of the ventilator used.

By way of example, a starting timestamp $T_{Ns}$ may be set as the starting time for the Nth time turning on the ventilator. An ending timestamp $T_{Ne}$ is set as the ending time for the Nth time turning off the ventilator. A log may comprise a plurality of starting and ending timestamps, each Nth starting timestamp being associated with an Nth ending timestamp. The total usage time in a day ($T_{total}$) can be calculated by adding all the used time using one or more of said logs.

The target usage time in a day ($T_{target}$) may be set by a respiratory caregiver or clinician, e.g., together with the ventilated subject. A reasonable target usage time would be around 5 hours per day. The recommended remaining ventilation time $T_{remaming}$ can be calculated as follows:

$$T_{remaining} = T_{target} - T_{total} = T_{target} - \sum_{N}^{M}(T_{Ns} - T_{Ne}) \qquad (3)$$

where M is the number of starting and/or ending timestamps in the log(s) for the ventilated subject for the (current) 24 hour period.

As previously explained, the number and/or size of icons provided by a visual representation of the recommended remaining ventilation time may indicate the length of the recommended remaining ventilation time.

For instance, each icon may represent a predetermined period of time. As a working example, the number of (full-sized) icons may indicate how many hours are still needed to complete the ventilation treatment. Thus, an absence of icons indicates that the ventilation treatment is complete. One of the icons may be modified in size, e.g., proportionally shrunk, to indicate the length of an hour left. Thus, if there are X hours and Y minutes remaining, where X and Y are non-zero, then X + 1 icons may be displayed with one of the icons having a size that is Z% the size of any other icon (where Z = Y/60).

Assuming that the time remaining is defined in minutes, the number of icons $Icon_{number}$ can be calculated as follows:

$$Icon_{number} = INT\left(\frac{T_{remaining}}{60}\right) \qquad (4)$$

where Int(.) is a rounding up function to the nearest higher integer. Equation (4) can be readily adapted for other approaches for defining time (e.g., if time is defined in seconds or the like).

The size of one of the icons $Icon_{size}$ may be modified using the following equation, again assuming that the time remaining is defined in minutes:

$$Icon_{size} = \left(1 + \frac{T_{remaining}}{60} - NT\left(\frac{T_{remaining}}{60}\right)\right) * Icon_{os} \qquad (5)$$

where $Icon_{os}$ is the original size of the icon.

In the context of the present disclosure, a size of the icon may represent a total area occupied by the icon. Reducing the size of an icon may comprise proportionally reducing the height and width of the icon (e.g., keeping it a fixed ratio) and/or deleting a proportional part of the icon.

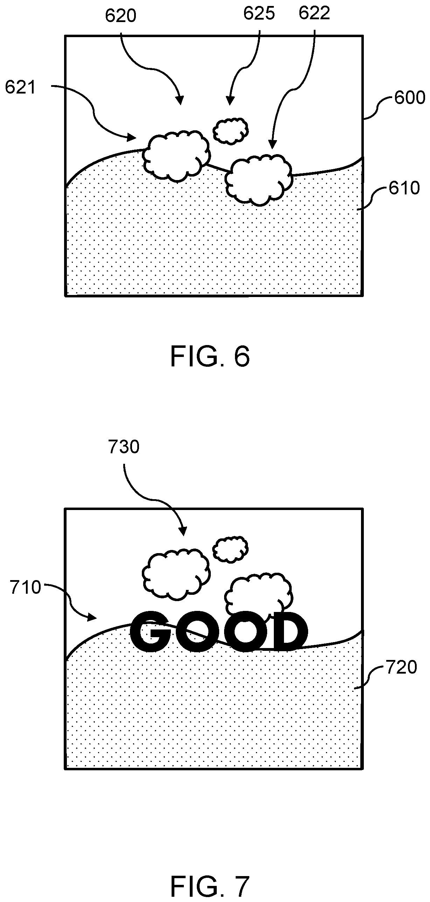

Fig. 6 conceptually illustrates an example visual representation 620 on a display 600 representing a recommended remaining ventilation time. Here, the example visual representation 620 is a second visual representation that partially overlays a first visual representation 610 of the display. The first visual representation 610 again indicates a value for a first ventilation parameter, e.g., humidity.

The number of full-sized icons 621, 622 (here: clouds) indicates the number of full hours left for recommended ventilation treatment. The size of an additional icon 625 indicates the proportion of another hour left for recommended ventilation treatment. In the illustrated example, the additional icon 625 is half the size of the full-sized icons, indicating half an hour. In total, the second visual representation thereby indicates that 2.5 hours of further ventilation are recommended. Of course, the additional icon 625 may be omitted if the number of hours remaining is an integer.

The proposed display method improves efficiency of understanding, because using the number and size of the icons to represent the remaining hours of treatment that day instead of the status of completed hours of treatment avoids the need for the operator of the ventilation system to learn the required hours and calculate hours by themselves by further interactions.

Another example of a ventilation parameter is an apnea hypopnea index. Accordingly, in some examples, the first ventilation parameter or the second ventilation parameter is an apnea hypopnea index. The apnea hypopnea index (AHI) is the number of apneas or hypopneas recorded during per hour of sleep. Generally, apneas (pauses in breathing) must last for at least 10 seconds and be associated with a decrease in blood oxygenation to contribute to the AHI.

The occurrence of an apnea event can be recorded by the built-in software of a ventilation system during sleep of the ventilated subject. The AHI can be trivially calculated by dividing the number of apnea events (preferably: meeting one or more apnea conditions) by the number of hours of sleep.

A visual representation for an AHI may, for instance, comprise an icon that changes responsive to the value of the AHI. In particular, the visual representation may change dependent upon in which of a set of predetermined ranges the value of the AHI falls.

By way of example, when AHI is below a first predetermined AHI (e.g., 5), the visual representation may be configured to show no icon. When AHI is between a first and second predetermined AHI (e.g., 5 <= AHI <= 15) an icon of an oxygen bottle may be shown with a (single) line on the body. This can remind or inform an operator that the medical subject has mild sleep apnea condition. When AHI is between a second and third predetermined AHI (e.g., 15 <= AHI <= 30) an icon of an oxygen bottle may be shown with two lines on the body. This can remind or inform an operator that the medical subject has a moderate sleep apnea condition. When AHI is greater than the third predetermined AHI (e.g., AHI >= 30) an icon of an oxygen bottle may be shown with three lines on the body, together with an optional additional alert icon (e.g., an exclamation line). This can remind or inform an operator that the medical subject has a moderate sleep apnea condition.

Fig. 7 conceptually illustrates another display 700 comprising another example of a visual representation 710 for another ventilation parameter.

In this example, the visual representation 710 indicates a value and/or indication for a determined status of the ventilation and/or the ventilated subject. In particular, the (other) ventilation information may be processed in order to identify or evaluate the status of the ventilation and/or ventilated subject. Thus, the status of the ventilation and/or ventilated subject may be determined and/or evaluated based on at least one ventilation parameter.

Approaches for performing such a procedure are known in the art, and may comprise determining which of a plurality of sets of predetermined conditions are met by the appropriate combination of at least one ventilation parameter. The value and/or indication for the determined status may depend upon which set of predetermined conditions is met by the ventilation information. More particularly, for example, different values and/or indications (e.g., "Excellent", "Good", "Fair", "Poor", "Bad") may be associated with a respective set of predetermined conditions.

The visual representation 710 of the value for the determined status may be positioned to at least partially overlap one or more other visual representations 720, 730 for a ventilation parameter. In one example, the visual representation 710 is arranged to overlap one or more other visual representations 720, 730, such that the visual representation 710 is sufficiently clear to the viewer (e.g., a clinician, caregiver or the ventilated subject) on the display 700 while the display for one or more other visual representations 720, 730 is not adversely influenced. In other wordings, one or more other visual representations 720, 730 are also sufficiently clear to the viewer (e.g., a clinician, caregiver or the ventilated subject) on the display 700. Thus, the visual representation of the determined status may act as a second visual representation in some embodiments.

Providing a visual representation of the (determined) status of ventilation and/or ventilated subject advantageously provides a viewer of the display with a general but quick prompt of the condition of the ventilation and/or subject.

The illustrated visual representation 710 of the value for the determined status here takes the form of a displayed word, although other examples will be apparent to the skilled person, e.g., an animation, a graph, a symbol (e.g., a thumb-up or thumb-down symbol) and so on.

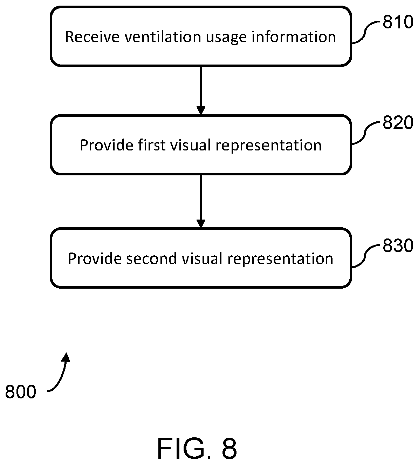

For the sake of completeness, Fig. 8 is a flowchart illustrating a computer-implemented method of controlling the display of ventilation information.

The computer-implemented method comprises a step 810 of receiving ventilation information comprising at least one value for each of at least two ventilation parameters for a subject undergoing mechanical ventilation, wherein the at least two ventilation parameters comprise a first ventilation parameter and a second ventilation parameter. The ventilation information may, for instance, be obtained from a ventilation system and/or memory communicatively coupled to the ventilation system.

The method 800 also comprises a step 820 of controlling a user interface to provide a first visual representation of the at least one value of the first ventilation parameter.

The method 800 also comprises a step 830 of controlling the user interface to provide a second visual representation of the at least one value of the second ventilation parameter, wherein the second visual representation at least partially overlays the first visual representation.

The method 800 may be adapted to control the first and second visual representations according to any previously described approach. Of course, the method may be adapted to control additional visual representations (e.g., of other ventilation parameters) where relevant.

With the proposed method 800 and the embodiments above, the ventilation parameters are better visualized to at least achieve a better interpretation with clear data timeliness, efficient data access, noticeable alerts and clear behavioral guidance, so as to promote correct usage and better compliance. For example, with the proposed visualization method, both patients and clinician can detect any issues (e.g., usage problems, patient status) more easily and straightforwardly and form an action plan by following the troubleshooting guidance, for example. Consequently, both patient and clinician are able to adjust their using behavior immediately for correct usage and better compliance. What's more, one patient can also make right overview of his patient status and ventilation status and make right and comply with follow-up actions, even though the patient has less or limited clinical experience and knowledge.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method (800) of controlling the display of ventilation information, the computer-implemented method comprising:

   receiving (810) ventilation information comprising at least one value for each of at least two ventilation parameters for a subject undergoing mechanical ventilation, wherein the at least two ventilation parameters comprise a first ventilation parameter and a second ventilation parameter;
   controlling (820) a user interface (110) to provide a first visual representation (210, 310, 410, 610, 720, 730) of the at least one value of the first ventilation parameter; and
   controlling (830) the user interface to provide a second visual representation (220, 320, 420, 620, 710) of the at least one value of the second ventilation parameter, wherein the second visual representation at least partially overlays the first visual representation.

2. The computer-implemented method of claim 1, wherein the second visual representation wholly overlays the first visual representation.

3. The computer-implemented method of any of claims 1 or 2, wherein:

   a textural appearance of the first visual representation changes as the at least one value for the first ventilation parameter changes; and/or
   a textural appearance of the second visual representation changes as the at least one value for the second ventilation parameter changes.

4. The computer-implemented method of any of claims 1 to 3, wherein the first visual representation and/or the second visual representation is an animated visual representation.

5. The computer-implemented method of claim 4, wherein:

   the at least two ventilation parameters further comprise a third ventilation parameter;
   the second visual representation is configured to provide a visual representation of the at least one value of the second ventilation parameter and the at least one value of the third ventilation parameter.

6. The computer-implemented method of claim 5, wherein the second visual representation is configured to sequentially provide the visual representation of the at least one value of the second ventilation parameter and the at least one value of the third ventilation parameter, such that the visual representation of the at least one value of the second ventilation parameter is not provided at a same time as the visual representation of the at least one value of the third ventilation parameter.

7. The computer-implemented method of any of claims 1 to 6, wherein:

   the first ventilation parameter or the second ventilation parameter is a time remaining ventilation parameter having a value that indicates a recommended remaining ventilation time for the subject, such that one of the first or second visual representation is associated with time remaining ventilation parameter; and
   one or more visual properties of the visual representation associated with the time remaining ventilation parameter changes responsive to the value that indicates a recommended remaining ventilation time for the subject.

8. The computer-implemented method of claim 7, wherein the one or more visual properties comprises one or more of: a transparency; a texture; a color; a size; and/or a number of icons.

9. The computer-implemented method of claim 8, wherein the one or more visual properties comprises a size of the visual representation, wherein the size of the visual representation associated with the time remaining ventilation parameter decreases proportionally with a decrease in the value of the recommended remaining ventilation time for the subject.

10. The computer-implemented method of any of claims 1 to 9, wherein:

the ventilation information further comprises, for the first and/or second ventilation parameter, time information identifying a time elapsed since a last recorded value for said ventilation parameter, and

the computer-implemented method further comprises:

when the time information identifies a time elapsed since the last recorded value for the first ventilation parameter, controlling a visual property of the first visual representation responsive to the time information; and/or

when the time information identifies a time elapsed since the last recorded value for the second ventilation parameter, controlling a visual property of the second visual representation responsive to the time information.

11. The computer-implemented method of claim 10, comprising:

when the time information identifies a time elapsed since the last recorded value for the first ventilation parameter, increasing a transparency of the first visual representation as the time elapsed since a last recorded value for the first ventilation parameter increases; and/or

when the time information identifies a time elapsed since the last recorded value for the second ventilation parameter, increasing a transparency of the second visual representation as the time elapsed since a last recorded value for the second ventilation parameter increases.

12. The computer-implemented method of any of claims 1 to 11, wherein:

the at least one value for the first ventilation parameter comprises a numerical measure of the first ventilation parameter; and

a hue angle of a color of the first visual representation changes proportionally to the numerical measure of the first ventilation parameter.

13. The computer-implemented method of any of claims 1 to 12, wherein at least one of the first visual representation and second visual representation is interactive, and

the computer-implemented method comprises, responsive to a user interaction with the first visual representation and/or the second visual representation:

identifying the first and/or second visual representation with which the user has interacted as an interacted visual representation; and

providing a further visual representation of at least one:

an explanation of the ventilation parameter associated with the interacted visual representation;

trouble-shooting guidance for the ventilation parameter associated with the interacted visual representation;

usage guidance for the ventilation parameter associated with the interacted visual representation; and/or

historic parameter information for the ventilation parameter associated with the interacted visual representation.

14. A computer program product comprising computer program code means which, when executed on a computing device having a processing system, cause the processing system to perform all of the steps of the method according to any of claims 1 to 13.

15. A user interface system comprising:

a user interface; and

a processing system configured to:

receive ventilation information comprising at least one value for each of at least two ventilation parameters for a subject undergoing mechanical ventilation, wherein the at least two ventilation parameters comprise a first ventilation parameter and a second ventilation parameter;

control the user interface to provide a first visual representation of the at least one value of the first ventilation parameter; and

control the user interface to provide a second visual representation of the at least one value of the second ventilation parameter, wherein the second visual representation at least partially overlays the first visual

representation.

FIG. 1

FIG. 2

320

300

85%

310

320

300

310

FIG. 3

FIG. 4

FIG. 5

FIG. 6

FIG. 7

Receive ventilation usage information — 810

Provide first visual representation — 820

Provide second visual representation — 830

800

# FIG. 8

## EUROPEAN SEARCH REPORT

Application Number

EP 23 20 4906

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "SERVO-U Ventilator System v4.0 – User's Manual", , 31 December 2019 (2019-12-31), pages 1-237, XP093141775, Retrieved from the Internet: URL:https://www.getinge.com/dam/hospital/documents/english/servo-u_4.0_user_manual-en-non_us.pdf [retrieved on 2024-03-15] * The whole document, in particular: Pages 6, 30, 31, 50 – 53 55, 57, 59, 60. * ----- | 1-15 | INV. G16H40/63 A61M16/00 G16H20/40 |
| X | US 2023/245768 A1 (NELLIS PATRICK J [CA] ET AL) 3 August 2023 (2023-08-03) * The whole document, in particular: Paragraphs [0009], [0025], [0033], [0052], [0056], [0066], [0068], [0076], [0077]; Figures 1, 2, 3A, 3C, 4, 5; Claim 1. * ----- | 1-15 | |
| X | US 2020/230336 A1 (ANGELICO PHYLLIS RAE [US]) 23 July 2020 (2020-07-23) * The whole document, in particular: Paragraphs [0004] – [0007], [0040] – [0060], [0063], [0067], [0071]; Figures 1, 2, 3a, 3b, 4a, 4b, 4c, 5, 6, 7; Claims 1, 2, 6, 11. * ----- | 1-15 | TECHNICAL FIELDS SEARCHED (IPC)  G16H A61M |

The present search report has been drawn up for all claims

| Place of search | Date of completion of the search | Examiner |
|---|---|---|
| Munich | 18 March 2024 | Ruschke, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 4906

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2024

| Patent document cited in search report | Publication date | Patent family member(s) | | Publication date |
|---|---|---|---|---|
| US 2023245768 A1 | 03-08-2023 | NONE | | |
| US 2020230336 A1 | 23-07-2020 | US 2020230336 A1 | | 23-07-2020 |
| | | WO 2020150404 A1 | | 23-07-2020 |

EPO FORM P0459

For more details about this annex : see Official Journal of the European Patent Office, No. 12/82